# EUROPEAN PATENT APPLICATION

(11) **EP 2 263 799 A2**
(43) Date of publication of application: **22.12.2010**
(21) Application number: 10183972.8
(22) Date of filing: 17.09.2001
(51) Int. Cl.: B01L 3/00

(54) **Microfabricated reaction chamber system**

(30) Priority: 15.09.2000 GB 0022754
(62) Divisional of application: 01967495.1
(71) Applicant: Norchip A/S, 3490 Klokkarstua (NO)
(72) Inventor: Karlsen, Frank, N-3490, Klokkarstua (NO)
(74) Representative: White, Nina Louise

(57) **Abstract**

A microfabricated reaction chamber system for carrying out a nucleic acid sequence amplification and detection process on a nucleic acid sample, the process comprising at least first and second process steps using first and second reagents, the device comprising:
an inlet port;
a first reaction chamber in communication with the inlet port, for carrying out the first process step;
a second reaction chamber in communication with the first reaction chamber, for carrying out the second process step; and
an outlet port in communication with the second reaction chamber.

## Description

The present invention relates to a microfabricated reaction chamber system for carrying out a nucleic acid sequence amplification and detection process on a nucleic acid sample, and in particular, but not exclusively to such a system for use in carrying out NASBA amplification and fluorescent molecular beacon probe detection process steps.

By the term microfabricated device or system as used herein is meant any device manufactured using processes that are typically, but not exclusively, used for batch production of semiconductor microelectronic devices, and in recent years, for the production of semiconductor micromechanical devices. Such microfabrication technologies include, for example, epitaxial growth (eg vapour phase, liquid phase, molecular beam, metal organic chemical vapour deposition), lithography (eg photo-, electron beam-, x-ray, ion beam-), etching (eg chemical, gas phase, plasma), electrodeposition, sputtering, diffusion doping and ion implantation. Although non-crystalline materials such as glass may be used, microfabricated devices are typically formed on crystalline semiconductor substrates such as silicon or gallium arsenide, with the advantage that electronic circuitry may be integrated into the system by the use of conventional integrated circuit fabrication techniques. Combinations of a microfabricated component with one or more other elements such as a glass plate or a complementary microfabricated element are frequently used and intended to fall within the scope of the term microfabricated used herein.

The isolation and purification of DNA and/or RNA from bacterial cells and virus particles is a key step in many areas of technology such as, for example, diagnostics, environmental monitoring, forensics and molecular biology research.

Microfabrication is an attractive construction method for producing devices for carrying out biological processes for which very small sample volumes are desirable, such as DNA sequence analysis and detection.

One such device, for carrying out a polymerase chain reaction (PCR) followed by a detection step is disclosed in US 5,674,742. Lamb wave pumps are used to transport DNA primers, polymerase reagents and nucleotide reagents from three separate storage chambers into a single reaction chamber as and when required to carry out a PCR process, with the temperature of the reaction chamber being cycled as required.

Another microfabricated device, for carrying out a chemical reaction step followed by an electrophoresis separation step, is disclosed in Analytical Chemistry 1994, 66, 4127-4132. Etched structures in a silicon substrate covered by a glass plate provide a reaction chamber and connections to buffer, analyte, reagent and analyte waste reservoirs, as well as an electrophoresis column connected to a waste reservoir.

Nucleic acid sequence-based amplification (NASBA) is a primer-dependent technology that can be used for the continuous amplification of nucleic acids in a single mixture at one temperature (isothermal nucleic acid amplification method) and was one of the first RNA transcription-based amplification methods described. NASBA normally offers a simple and rapid alternative to PCR for nucleic acid amplification, and is capable of yielding an RNA amplification of a billion fold in 90 minutes. With respect to other amplification systems such as the PCR technique, the ability of NASBA to homogeneously and isothermally amplify RNA analytes extends its application range from viral diagnostics to the indication of biological activities such as gene expression and cell viability. NASBA technology is discussed, for example, in Nature volume 350 pages 91 and 92.

Nucleic acid amplification in NASBA is accomplished by the concerted enzyme activities of AMV reverse transcriptase, Rnase H, and T7 RNA polymerase, together with a primer pair, resulting in the accumulation of mainly single-stranded RNA that can readily be used for detection by hybridization methods. The application of an internal RNA standard to NASBA results in a quantitative nucleic acid detection method with a dynamic range of four logs but which needed six amplification reactions per quantification. This method is improved dramatically by the application of multiple, distinguishable, internal RNA standards added in different amounts and by electrochemiluminesence (ECL) detection technology. This one-tube quantitative (Q) NASBA needs only one step of the amplification process per quantification and enables the addition of the internal standards to the clinical sample in a lysis buffer prior to the actual isolation of the nucleic acid. This approach has the advantage that the nucleic acid isolation efficiency has no influence on the outcome of the quantitation, which in contrast to methods in which the internal standards are mixed with the wild-type nucleic acid after its isolation from the clinical sample. Quantitative NASBA is discussed in Nucleic Acid Research (1998) volume 26, pages 2150-2155.

Post-NASBA product detection, however, can still be a labour-intensive procedure, normally involving enzymatic bead-based detection and electrochemiluminescent (ECL) detection or fluorescent correlation spectrophotometry. However, as these methodologies are heterogeneous or they require some handling of sample or robotic devices that are currently not cost-effective they are relatively little used for high-throughput applications. A homogeneous procedure in which product detection is concurrent with target amplification by the generation of a target-specific signal would facilitate large-scale screening and full automation. Recently, a novel nucleic acid detection technology, based on probes (molecular beacons) that fluoresce only upon hybridization with their target, has been introduced.

Molecular beacons are single-stranded oligonuclotides having a stem-loop structure. The loop portion contains a sequence complementary to the target nucleic acid, whereas the stem is unrelated to the target and has a double-stranded structure. One arm of the stem is labelled with a fluorescent dye, and the other arm is labelled with a non-fluorescent quencher. In an isolated state the probe does not produce fluorescence because absorbed energy is transferred to the quencher and released as heat. When the molecular beacon hybridizes to its target it undergoes a conformational change that separates the fluorophore and the quencher, and the bound probe fluoresces brightly. Molecular beacon probes are discussed, for example, in US 6,037,130 and in Nucleic Acids Research, 1998, vol. 26, no.9.

Even the one tube quantitative Q-NASBA process generally requires at least two steps, typically a first primer annealing step carried out at about 65 degrees Celsius followed by an amplification and detection step carried out at about 41 degrees Celsius. The enzymes required for the second step would be denatured by the elevated temperature required for the first step, so must be added once the temperature of the process components has fallen sufficiently. Furthermore, as for most nucleic acid sequence amplification and detection processes, NASBA requires reagents specific to the target nucleic acid sequence to be used. To carry out simultaneous analysis of a DNA/RNA sample for a number of different target nucleic acid sequences generally requires the handling of a large number of different reagent sets, each requiring separate handling and use in separate test tubes.

The present invention seeks to address at least some of the problems of the prior art.

Accordingly, the present invention provides a microfabricated reaction chamber system for carrying out a nucleic acid sequence amplification and detection process on a nucleic acid sample, the process comprising at least first and second process steps using first and second reagents, the device comprising:
an inlet port;
a first reaction chamber in communication with the inlet port, for carrying out the first process step;
a second reaction chamber in communication with the first reaction chamber, for carrying out the second process step; and
an outlet port in communication with the second reaction chamber.

A single fluid path, whereby the sample is passed into the first reaction chamber for carrying out the first process step, then into the reaction second chamber for the second process step, simplifies construction and operation of the device.
By providing two separate reaction chambers, separate reagents for separate process steps may be conveniently preloaded into the system before use, and no active components are then required to move reagents within the device during use. The reagents for second and any subsequent process steps may be protected from damaging environmental conditions such as elevated temperatures required during earlier process steps.

A sample loading chamber may be provided between the inlet port and the first reaction chamber, for example to facilitate controlled loading of a sample into the device or first reaction chamber.

The first reagent (which may be substantially dry) is preferably preloaded within the first reaction chamber and the second reagent (which may be substantially dry) is preferably preloaded within the second reaction chamber. This may conveniently be carried out during manufacture of the system. To enhance storage qualities the reagent mixes may be lyophilised or dried *in situ.*

The nucleic acid sample may be derived from, for example, a biological fluid, a dairy product, an environmental fluids and/or drinking water. Examples include blood, serum, saliva, urine, milk, drinking water, marine water and pond water. For many complicated biological samples such as, for example, blood and milk, it will be appreciated that before one can isolate and purify DNA and/or RNA from bacterial cells and virus particles in a sample, it is first necessary to separate the virus particles and bacterial cells from the other particles in sample. It will also be appreciated that it may be necessary to perform additional sample preparation steps in order to concentrate the bacterial cells and virus particles, i.e. to reduce the volume of starting material, before proceeding to break down the bacterial cell wall or virus protein coating and isolate nucleic acids. This is important when the starting material consists of a large volume, for example an aqueous solution containing relatively few bacterial cells or virus particles. This type of starting material is commonly encountered in environmental testing applications such as the routine monitoring of bacterial contamination in drinking water.

The system is preferably designed to cater for a sample volume of ≤ 50 nl, preferably ≤ 20 nl, more preferably ≤ 10 nl. Thus the volume of each of the reaction chambers will typically be ≤ 500 nl, more typically ≤ 300 nl, still more typically ≤ 150 nl.

Preferably, one or more temperature controllers are provided to enable the first process step to be carried out at a first temperature, and to enable the second process step to be carried out at a second temperature that is lower than the first temperature. For example, separate first and second temperature controllers may be used to control the temperatures of the first and second process steps. Various nucleic acid amplification processes, such as NASBA processes, which require higher temperatures for an earlier process step, which would denature reagents used in a later process steps, may in this way be conveniently and easily carried out in a microfabricated reaction chamber system or device.

Preferably, means are provided for heating the contents of the first chamber to a temperature of from 60 to 70°C, more preferably from 63 to 67°C, still more preferably ≈ 65°C. Preferably, means are provided for heating the contents of the second chamber to a temperature of up to 41.5°C, more preferably ≈ 41°C.

Preferably, the first temperature controller comprises a first temperature sensor positioned adjacent to the first reaction chamber and the second temperature controller comprises a second temperature sensor positioned adjacent to the second reaction chamber.

Preferably, the first temperature controller comprises a first controllable electric heat source (for example an electrical resistor element) positioned adjacent to the first reaction chamber and the second temperature controller comprises a second controllable electric heat source (for example an electrical resistor element) positioned adjacent to the second reaction chamber.

The system may thus preferably include integrated electrical heaters and temperature control.

Peltier element(s) and/or thermocouple(s) may be used to maintain the sample at the desired temperature in the first and/or second chambers, preferably to within ± 0.5 °C. In particular, thermocouples may be used to measure the temperature of the first and second chambers, wherein the thermocouples are linked by one or more feedback circuits to Peltier elements for heating the sample to the desired temperature in the first and second chambers.

A thermal barrier may advantageously be provided the substantially thermally isolate the second chamber from the first chamber.

The outlet port may be provided with a pressure control valve or flow control valve, to control the flow of the sample and/or other fluids through the system. This may be used in conjunction with a pump, which may be provided at the inlet end of the system, to control the flow of fluids into and through the reaction chambers.

The system may be provided with an optical interface for excitation and/or detection purposes. Accordingly, if optical observations of the contents of the second reaction chamber are required, then at least one wall defining the second chamber comprises an optically transparent substance or material, for example glass.

Preferably, the system comprises at least one optical source arranged for exciting fluorescence in material contained within the second reaction chamber, and at least one optical detector, arranged to detect said fluorescence. For example, molecular beacon probes may be provided in the second reaction chamber to detect one or more target nucleic acid sequences. These probes fluoresce when in the presence of target nucleic acid sequences, thereby enabling detection and quantification of such sequences. The system thus provided amplification combined with optical-fluorescence detection. However, other detection methods could be used, for example, using impedance measurements.

Preferably, the optical source is provided by one or more light emitting diodes, and the optical detector comprises at least one avalanche photodiode. However, other sources and detectors could also be used. For example the optical detector could comprise at least one photomultiplier tube.

Preferably, a bandpass filter is provided to filter the light impinging on the detector, in particular to filter out light emitted by the optical source.

A micro-lens may be provided to direct the fluorescence onto the detector or, alternatively, a confocal microscope could be used.

An integrated microfabricated reaction chamber system may be provided with a plurality of sets interconnected first and second reaction chambers as described above, each of which may have a separate outlet port. In this way a range of different analysis processes may be carried out simultaneously within a single micromachined device.

A single set of first and second reaction chambers, or multiple sets of first and second reaction chambers may be connected to a common inlet port. Such an inlet port may advantageously be provided with a micro pump to pass the sample into the reaction chambers.

The system will typically be formed from or comprise a semiconductor material, although dielectric (eg glass, fused silica, quartz, polymeric materials and ceramic materials) and/or metallic materials may also be used. Examples of semiconductor materials include one or more of: Group IV elements (i.e. silicon and germanium); Group III-V compounds (eg gallium arsenide, gallium phosphide, gallium antimonide, indium phosphide, indium arsenide, aluminium arsenide and aluminium antimonide); Group II-VI compounds (eg cadmium sulphide, cadmium selenide, zinc sulphide, zinc selenide); and Group IV-VI compounds (eg lead sulphide, lead selenide, lead telluride, tin telluride). Silicon and gallium arsenide are preferred semiconductor materials. The system may be fabricated using conventional processes associated traditionally with batch production of semiconductor microelectronic devices, and in recent years, the production of semiconductor micromechanical devices. Such microfabrication technologies include, for example, epitaxial growth (eg vapour phase, liquid phase, molecular beam, metal organic chemical vapour deposition), lithography (eg photo-, electron beam-, x-ray, ion beam-), etching (eg chemical, gas phase, plasma), electrodeposition, sputtering, diffusion doping and ion implantation. Although non-crystalline materials such as glass and polymeric materials may be used, the microfabricated system is usually formed on crystalline semiconductor substrates such as, for example, silicon or gallium arsenide, with the advantage that electronic circuitry may be integrated into the system by the use of conventional integrated circuit fabrication techniques. Combinations of a microfabricated component with one or more other elements such as a glass plate or a complementary microfabricated element are frequently used and intended to fall within the scope of the term microfabricated used herein.

Examples of polymeric materials include hydrophilic polymers in order to prevent unspecific binding of cells and proteins. Suitable examples include PMMA (Polymethyl methylacrylate), COC (Cyclo olefin copolymer), PL (Polylactide), PBT (Polybutylene terephthalate) and PSU (Polysulfone). The system may be formed by plastic replication of, for example, a silicon master.

The one or more systems will typically be integrally formed. The systems may be microfabricated on a common substrate material, preferably a semiconductor material, although a dielectric substrate material such as, for example, glass or a ceramic material could be used.

The microfabricated system may be designed to be disposable after it has been used once or for a limited number of times. This is an important feature because it reduces the risk of contamination.

The microfabricated system may be incorporated into an apparatus for the analysis of, for example, biological fluids, dairy products, environmental fluids and/or drinking water. Again, the apparatus may be designed to be disposable after it has been used once or for a limited number of times.

The microfabricated system/apparatus may be included in an assay kit for the analysis of, for example, biological fluids, dairy products, environmental fluids and/or drinking water, the kit further comprising means for contacting the sample with the device. Again, the assay kit may be designed to be disposable after it has been used once or for a limited number of times.

The microfabricated system as herein described is also intended to encompass nanofabricated devices.

In a preferred embodiment, the system described above is formed from a silicon or silicon-comprising substrate (although other of the above recited materials may be used), capped with a top plate, which may be made of glass (for example Pyrex), each reaction chamber being defined by a recess in a surface of the substrate and the adjacent surface of the top plate. A channel is provided between the reaction chambers. Preferably, and in particular if optical observations of the contents of the second reaction chamber are required, the top plate may be made of glass (for example Pyrex) or another optically transparent substance or material. The combination of silicon and glass provides suitable thermal, surface and optical properties, as well as adequate surface properties. It is possible to define by, for example, etching microfluidic channels, reaction chambers and fluid interconnects in the silicon substrate with accurate microscale dimensions. In this manner a silicon wafer with an etched microstructure may be anodically bonding it to a pyrex wafer thereby forming the enclosed chambers and channels. The glass wafer allows the reagents to be optically excited and detected through the glass. The silicon wafer enables electronic components to be integrated.

If the system includes an optical source arranged for exciting fluorescence in material contained within the second reaction chamber, and an optical detector, arranged to detect said fluorescence, the surface in the second reaction chamber is preferably optically smooth. It has been found that the surface roughness of the wall(s) defining the second chamber on which light may be incident should be less than approximately 1/10th of the wavelength of the light. Reactive-Ion-Etching (RIE) of said wall(s) of the second chamber has been found to achieve a good surface quality with a roughness of less than approximately 40 nm.

Different regions of a channel may define the an inlet port and the first reaction chamber. Similarly, different regions of a channel may define the an the second reaction chamber and the outlet port.

The present invention also provides a microfabricated reaction chamber system for carrying out a nucleic acid sequence amplification and detection process on a nucleic acid sample in a single reaction chamber, the device comprising:
a reaction chamber having an inlet port and an outlet port and at least one wall that comprises an optically transparent material;
means for heating a sample contained the reaction chamber to a temperature of ≤ 41.5°C (preferably from 40.5 to 41.5°C);
at least one optical source arranged adjacent said at least one wall for exciting fluorescence in a sample contained in the reaction chamber; and
at least one optical detector, arranged to detect said fluorescence through said at least one wall.

Such a device dispenses with the first chamber and may be used in circumstances where the target does not require a pre-denaturing step. Real-time amplification and detection takes place in a single microfabricated reaction chamber. The features (for example materials and construction) described herein in relation to the two chamber system are also pertinent and relevant to the single chamber system.

The present invention also provides a device which includes a microfabricated reaction chamber system as herein described, together and preferably in fluid communication with one or more of: (i) means for filtering a sample prior to carrying out the method according to the present invention, for example to substantially remove particles contained in the sample which are larger in size that bacteria particles; and/or
(ii) means for separating virus particles and/or bacterial cells from the other particles in a sample prior to carrying out the method according to the present invention; and/or
(iii) means for concentrating bacterial cells and/or virus particles, i.e. to reduce the volume of starting material, prior to carrying out the method according to the present invention; and/or
(iv) means for breaking down the bacterial cell wall or virus protein coating and isolate nucleic acids prior to carrying out the method according to the present invention.

The present invention also provides a method of carrying out a nucleic acid sequence amplification and detection process on a nucleic acid sample in a microfabricated reaction chamber system, the process comprising at least a first process step using a first reagent and a second process step using a second reagent, the method comprising the steps of:
passing the sample into a first reaction chamber and mixing the sample with the first reagent;
retaining the sample in the first reaction chamber for a first interval to carry out the first process step;
passing the sample from the first reaction chamber into the second reaction chamber and mixing the sample with the second reagent;
retaining the sample in the second reaction chamber for a second interval to carry out the second process step; and
during the second interval, measuring a physical phenomenum indicative of the presence of the target nucleic acid sequence.

The method is particularly advantageous when the process is a NASBA or quantitative NASBA process. The first and second reagents may comprise NASBA primers, ribonucleoside and deoxyribonucleoside triphosphates, enzymes for carrying out a NASBA reaction and molecular beacon probe oligonucleotide.

Preferably, the method further comprises the steps of maintaining the contents of the first reaction chamber at a first temperature during the first interval and maintaining the contents of the second reaction chamber at a second temperature during the second interval, wherein the first temperature is higher than the second temperature, and is sufficiently high to denature the second reagent. The first temperature is typically from 60 to 70°C, preferably from 63 to 67°C, more preferably ≈ 65°C. The second temperature is typically 35 to 45°C, preferably ≤≈ 41.5°C, more (preferably ≈ 41°C.

Preferably, the step of measuring comprises the steps of irradiating the second chamber to excite fluorescence and detecting any such fluorescence, for example in molecular beacon probes present in the second chamber which are adapted to fluoresce on annealing with a target nucleic acid sequence.

The method preferably uses the microfabricated reaction chamber system as herein described. In circumstances where the target does not require a pre-denaturing step, then a single chamber system as herein described may be used. In that case, the method may comprise:
assembling a reaction mixture in the single chamber, said mixture comprising the nucleic acid sample, NASBA primers, enzymes for carrying out a NASBA reaction, ribonucleoside and deoxyribonucleoside triphosphates, and molecular beacon probe oligonucleotide;
heating said mixture contained in said reaction chamber to a temperature of ≤ 41.5°C (preferably from 40.5 to 41.5°C);
exciting fluorescence in said mixture contained in said reaction chamber by means of said at least one optical source; and
detecting fluorescence in said mixture through said at least one wall by means of said at least one optical detector.

It will be appreciated that one or more of the NASBA primers, enzymes for carrying out a NASBA reaction, ribonucleoside and deoxyribonucleoside triphosphates, and molecular beacon probe oligonucleotide may be preloaded in the single chamber. Alternatively, or in combination with partial pre-loading, one or more of the aforementioned reactants may be mixed with the sample prior to passing it to the chamber.

A number of embodiments of the invention will now be described, with reference to the accompanying drawings, of which:
Figure 1 is a plan view of a microfabricated reaction chamber device according to a first preferred embodiment of the present invention, for carrying out a nucleic acid sequence amplification and detection process;
Figure 2 is a sectional view along line X-X of figure 1;
Figure 3 is a plan view of the lumen structure of a microfabricated reaction chamber device according to a second preferred embodiment of the invention;
Figure 4 is a plan view of the lumen structure of a microfabricated reaction chamber device according to a third preferred embodiment of the invention.

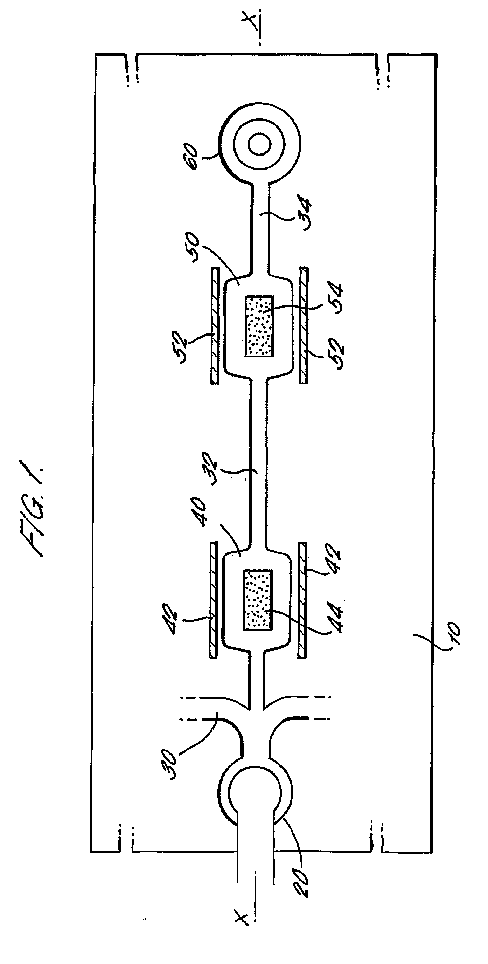

Referring to figure 1 there is shown a plan view of a microfabricated reaction chamber device 10 for carrying out first and second steps of a nucleic acid amplification and detection process. The device is provided with one or more fluid flow lumens consisting of channels and chambers for carrying a fluid sample introduced through an inlet port 20. The inlet port is connected to a supply channel system 30 which provides branches to one or more separate sets of reaction chambers, only one set of which is shown in figure 1. Each set of reaction chambers comprises a first reaction chamber 40 connected to the supply channel system 30, a transfer channel 32 connecting the first reaction chamber 40 and a second reaction chamber 50, and an exit channel 34 connected between the second reaction chamber and an outlet port 60.

A number of first heating elements 42 and a first temperature sensor 44 are provided adjacent to the first reaction chamber 40. These components are connected to control circuitry which enables the temperature of the walls and contents of the first reaction chamber to be carefully controlled. A number of second heating elements 52 and a second temperature sensor 54, along with suitable control circuitry, are similarly provided adjacent to the second reaction chamber.

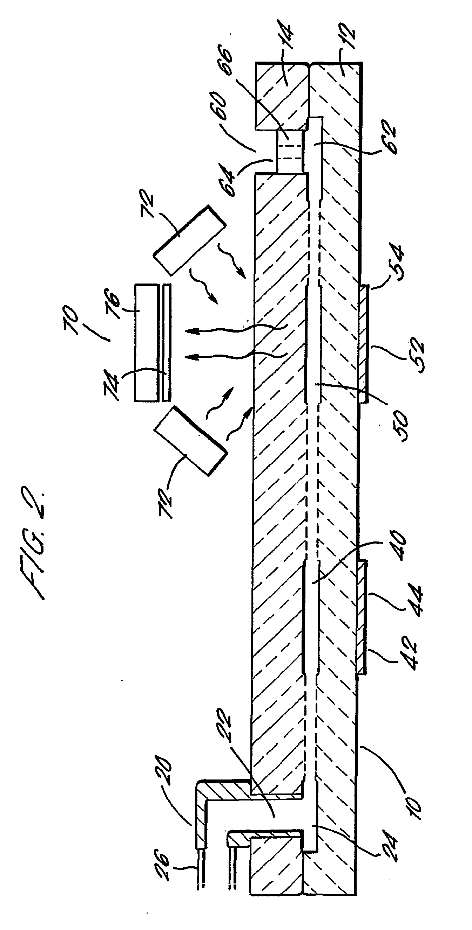

Turning now to figure 2, there is shown a sectional view of the micro reaction chamber device of figure 1 along line X--X. The device is constructed by etching the required channels and chambers from the surface of a silicon substrate 12. A glass top plate 14 is then anodically bonded to the surface of the silicon substrate 12 to enclose the channels and chambers. The top plate may advantageously be made of a borosilicate glass such as Pyrex (trademark), which has a low coefficient of thermal expansion.

The inlet port 20 comprises an inlet port chamber 24 etched into the substrate and overlain by an inlet port aperture 22 in the glass plate 14. An inlet port connector 26 is bonded in position over or accepted into the inlet port aperture 22. The inlet port may equally comprise a structure adapted to accept the tip of a micropipette or similar device.

The first 42 and second 52 heating elements are provided by electrical resistance elements bonded, printed, or otherwise provided on the lower surface of the silicon substrate, close to the appropriate reaction chamber. The first and second temperature sensors 44,54 are provided by, for example, thermistors or platinum resistors bonded, printed or otherwise constructed on the underside of the substrate directly underneath the appropriate reaction chamber.

The outlet port 60 is provided by an outlet port chamber 62 formed from an etched recess in the upper surface of the silicon substrate 12, an outlet port aperture 64 in the glass top plate directly above the outlet port chamber, and an outlet port valve 66 fitted into or on top of the outlet port aperture. The outlet port valve is electrically controllable to assist in controlling the flow of fluids through the reaction chambers, for example by allowing air or fluid flow out of the outlet port only when the pressure in the outlet port chamber 62 reaches or exceeds a certain controlled value.

An optical system 70 is provided to stimulate and detect fluorescence of particular materials contained within the second reaction chamber 50. Such an optical system may clearly be provided in a variety of ways, but in the embodiment shown in figure 2 there are provided two light emitting diodes (LEDs) 72 arranged to irradiate the contents of the second reaction chamber 50 through the glass plate, a cascade photodiode detector 76 for detecting fluorescence photons emitted by material in the second reaction chamber 50, and an optical bandpass filter 74 to shield the detector 76 at least from light having wavelengths generated by the LEDs 72. In the preferred embodiment one of the LEDs 72 emits light having a wavelength of about 488 nm, and the other LED emits light having a wavelength of about 590 nm. The optical bandpass filter 74 limits the wavelengths of light reaching the detector 76 to between 515 nm and 565 nm and above 590 nm.

The optical system is provided with suitable control circuitry to carry out the irradiation and detection process and to forward data regarding the detected fluorescence to data processing facilities.

During construction of the microfabricated reaction chamber device first and second reagents for carrying out first and second process steps are deposited in the first and second reaction chambers. This may be carried out before the glass top plate is bonded to the substrate, for example, by micro pipetting each reagent into the appropriate reaction chamber and, if required, carrying out a drying or lyophilisation procedure to reduce each reagent to a dry deposit. Alternatively, the reagents could be injected in fluid form through the inlet and outlet ports 20,60, and then lyophilised if required.

To use the device, a prepared sample is pumped through the inlet port 20 (for example by a micro pump) and into the first reaction chamber 40, where it mixes with the first reagent. The first reaction chamber is maintained at a desired temperature, or the temperature is varied in a desired time dependent manner, in order to carry out the first process step. By applying further pressure at the inlet port the sample is then pumped from the first reaction chamber 40 to the second reaction chamber 50 where it mixes with the second reagent. The second reaction chamber is then maintained at a desired temperature, or the temperature is varied in a desired time dependent manner in order to carry out the second process step. During the second process step the optical system is used to stimulate and detect fluorescence in the material contained within the second reaction chamber as required, in particular to obtain information about the processed sample.

Transport of fluids through the system may be controlled by controlling the pumping of the sample through the inlet port 20. Control of the pressure in or flow through the system by means of the control valve 66 in the outlet port 60 may also be used. If a single pumping device is used to feed a number of sets of parallel reaction chambers through supply channel system 30, individual pressure control valves 66 for each set of first and second reaction chambers may be used to ensure the correct fluid flow through each set of reaction chambers. Feedback data regarding the fluid flow may be obtained by detecting the presence of the sample in one or both reaction chambers, for example by detecting a fluorescence signal from the second reaction chamber.

Use of the described first embodiment of a microfabricated reaction chamber device for carrying out a Q-NASBA nucleic acid sequence amplification process, followed by detection of the target sequence by means of molecular beacon probes will now be described.

During or after manufacture of the microfabricated reaction chamber device, ten nanolitres of a first reagent are deposited in the first reaction chamber, which is approximately 50 x 300 x 100 micrometers in size. An appropriate corresponding cross sectional size for the various connecting channels is about 50x50 micrometers. The first reagent consists of about 0.5 nl of NASBA buffer and 0.5 nl of primer mix solution. The NASBA buffer consists of the final concentration of 20 micromolar molecular probes (specific towards the target RNA sequence), 100 nmol/nl of ROX (fluorescence control), 5 mM of each dNTP, 2 mM of ATP, UTP and CTP, 6.5 mM GTP, and 2.5 mM ITP, 2.5 mM dithiotreitol, 350 mM KCl, 60 mM MgCl₂, 200 mM Tris-HCl (pH 8.5). The primer mix solution consists of 45% DMSO and 0.2 microM each of the antisense and sense primers.

One nanolitre of a second reagent is deposited in the second reaction chamber, which is about the same size as the first reaction chamber. The second reagent consists of 1875 mM sorbitol, 12.5 microgram BSA, 0.4 U RNase H, 160 U T7 RNA polymerase and 32 U AMV-reverse transcriptase. The first and second reagents are then lyophilised.

A sample containing DNA/RNA is prepared, for example by culturing a cell sample, concentrating the cultured cell sample, carrying out a lysis step to liberate DNA and RNA from the cultured cells, and purifying the sample to selectively retain DNA/RNA fragments. About 9 nl of the prepared sample is then passed into the first reaction chamber where it mixes with the first reagent, and is held at 65 degrees Celsius for 5 minutes. The sample is then passed into the second reaction chamber and held there at 41 degrees Celsius for about 60 minutes, or until the fluorescence signal has reached an optimum level. During this time, each of the two LEDs is alternately switched on and off for a period of five seconds or more. The fluorescence detected by the detector during each 5 second interval is recorded in order to establish the quantity of the target nucleic acid sequence present in the second reaction chamber.

A number of variations in and alternatives to the first preferred embodiment will now be described. These variations also generally apply to the second and third preferred embodiments described below. Figures 1 and 2 show only a single set of reaction chambers, comprising two interconnected reaction chambers and an outlet port. For many purposes it may be desirable to provide two or more sets of reaction chambers in a single device. The plurality of sets of reaction chambers may be fed by a single inlet port connected to a branched supply channel system, or by multiple inlet ports. Each set of reaction chambers may be provided with a separate outlet port 66 having a pressure or flow control valve 66 to allow fluid flow through each individual system to be accurately controlled.

Although a set of reaction chambers having only first and second interconnected reaction chambers has been described in respect of the first preferred embodiment, clearly a similar reaction chamber system could comprise other combinations of reaction chambers with appropriate interconnections to carry out a variety of reaction and detection processes.

If a plurality of sets of reaction chambers are provided in a single device, then each set of reaction chambers can be charged with reagents to carry out a different analysis process. For example, each of many sets of reaction chambers could be provided with reagents to detect a different target nucleic acid sequence. In this way, a plurality of different nucleic acid sequences could be tested for simultaneously using one device preconfigured with all the necessary reagents.

Flow through the one or more sets of reaction chambers may be driven by a pump such as a syringe, rotary pump or precharged vacuum or pressure source external to the device. Alternatively, a micro pump or vacuum chamber, or lamb wave pumping elements could be provided as part of the device itself. Other combinations of flow control elements including pumps, valves and precharged vacuum and pressure chambers may clearly be used to control the flow of fluids through the reaction chambers. Electronic control circuitry will generally be required to control the operation of the active flow control elements used, such as pumps and valves. This control may be carried out, for example, on the basis of carefully timed operation of the active flow control elements, by using dedicated sensors to detect fluid presence or flow, or by obtaining feedback from the flow control elements themselves to determine the presence or location of pumped fluids. Other possible mechanisms for transporting fluids within the system include electroosmotic flow.

In the preferred embodiment described above the reagents are introduced into the reaction chambers during manufacture of the device and retained there until the device is used, for example in solid or liquid form. Clearly, the reagents could equally be held in other chambers ancillary to the main reaction chambers, and introduced when required into the appropriate reaction chambers.

To excite fluorescence in the material contained in the second reaction chamber, the preferred embodiment makes use of LEDs. These LEDs could be bonded or mounted to the upper surface of the glass top plate. Alternatively, they could be held in position by apparatus external to the microfabricated reaction chamber device itself. For a device having multiple sets of reaction chambers, one or more separate LEDs having the desired emission characteristics could be provided for each second reaction chamber, for particular groups of reaction chambers, or for the whole device. Other radiation sources could of course be used, for example discharge tube or laser sources, perhaps channelled to the appropriate reaction chambers by means of fibre optics.

To detect fluorescence, the preferred embodiment makes use of an avalanche photodiode provided with a filter to exclude light of unwanted frequencies. A lens structure may be provided, for example formed by a surface of the glass top plate or separately provided to assist in focussing the fluorescence photons towards the detector. Other detection systems may equally be used, for example a confocal microscope arrangement, perhaps scanning across multiple second reaction chambers, in combination with a photomultiplier tube detector.

Various physical phenomena could be used instead of or as well as fluorescence to determine the progress or results of reaction processes carried out in the second reaction chamber, or indeed in the first reaction chamber. For example, process steps may be used that exhibit particular impedance characteristics, detectable by a simple resistance measurement, or heating/cooling characteristics which could easily be measured using the temperature control components already described, possibly with some reference temperature detection elements located away from the reaction chamber concerned.

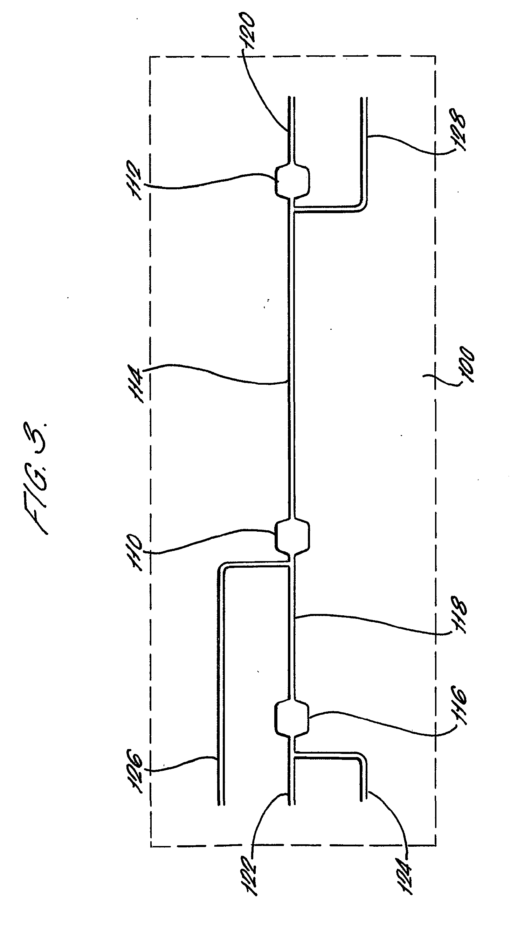

Referring now to figure 3 there is shown in plan view the fluid carrying lumens of a device according to a second embodiment of the invention. Other aspects of the second embodiment, such as temperature control apparatus, inlet and outlet ports, and illumination and fluoresence detection apparatus are not shown, but may be provided as already described above in respect of the first preferred embodiment.

The device 100 of the second embodiment is provided with first and second reaction chambers 110,112 interconnected by an intermediate channel 114. A loading chamber 116, of the same dimensions as each of the reaction chambers, is connected to the first reaction chamber 110 by a sample transfer channel 118. An exhaust channel to allow fluid or gas release 120 is connected to the second reaction chamber 112.

A sample loading channel 122 is connected to the loading chamber 116 to allow a DNA/RNA sample to be introduced into the loading chamber 116. A sample propulsion channel 124 connects to the sample loading channel, at a point adjacent to the loading chamber 116. A first reagent loading channel 126 connects to the sample transfer channel 118 at a point adjacent to the first reaction chamber 110, and a second reagent loading channel 128 connects to the intermediate channel 114 adjacent to the second reaction chamber 112.

The device 100 may be fabricated from an etched silicon wafer closed with a glass top plate. The dotted boundary in figure 3 shows a nominal boundary for the top plate.

A suitable channel depth for the device of the second preferred embodiment is 50 µm. A suitable volume for the reaction and loading chambers is about 10 nanolitres, which can be achieved using a chamber with lateral dimensions of about 450 µm x 450 µm and a depth of 50 µm, and by rounding the corners with a radius of curvature of 100 µm. Rounding the corners in this way reduces the chances of air pockets lodging in the chambers.

By making some parts of the fluid carrying lumens more hydrophobic or hydrophilic than other parts, fluid can be held preferentially in certain regions. For the present embodiment, silicon oxide, which can be grown in an oxygen plasma machine on a silicon substrate, is used to create hydrophilic regions, while hydrophobic regions are defined by etching away patterns of silicon oxide using standard lithographic techniques.

Regions which are treated to be relatively hydrophobic in the second preferred embodiment are shown as hatched regions in the lumens of figure 3. These regions are: along the sample propulsion channel 124 from the junction with the sample loading channel 122 for about 1000 µm; along the sample transfer channel 118 from the junction with the loading chamber 116 for about 100 µm and from the first reaction chamber for about 350 µm, along the first reagent loading channel 126 from the junction with the sample transfer channel 118 for about 100 µm; along the intermediate channel 114 from the junction with the first reaction chamber 110 for about 100 µm and from the junction with the second reaction chamber for about 100 µm; along the second reagent loading channel 128 from the junction with the intermediate channel for about 100 µm; and along the length of the exhaust channel.

Trenches may be formed in the silicon substrate in appropriate locations to help thermally isolate the first and second reaction chambers from each other and from other parts of the device.

A variety of methods and apparatus familiar to the person skilled in the art may be used to input and control the flow of sample fluid and reagents within the lumens of the second embodiment, including the methods and apparatus described above in respect of the first preferred embodiment. For example, fluid may be injected into a lumen of the device using a fine sequencing pipette tip guided into the end of one of the channels at the edge of the glass top plate by a trench formed at the end of the channel. An airtight and watertight seal may be made between the pipette tip and the channel using epoxy. Alternatively, a small hole, for example about 200 µm across, could be etched in the top plate centred on the end of a channel, and a pipette tip inserted into the hole and sealed if necessary.

Fluids may be manipulated using fine syringes capable of accurately delivering nanolitre volumes via the above-mentioned pipette tips into the device.

Reagents may be loaded into the first reaction chamber 110 by blocking the ends of all channels except the first reagent loading channel 126 and exhaust channel 120 and using a fine syringe connected to the first reagent loading channel 126 to inject the require volume of reagents into the first reaction chamber 110. The hydrophobic regions in the channels around the first reaction chamber 110 then encourage the reagents to stay in place. A similar procedure, wherein the ends of all channels except the second reagent loading channel 128 and the exhaust channel 120 are blocked off, can be used to load the second reagents into the second reaction chamber 112 where they are held in place by the hydrophobic regions surrounding that chamber.

The reagents, when loaded in the reaction chambers, may be dessicated by placing the device in a vacuum dessication chamber. A sample fluid may then be loaded into the sample loading chamber 116 by blocking off the ends of the first and second reagent loading channels 126,128, but leaving other channels open, and injecting 10 nl of sample fluid through the sample loading channel 122. The hydrophobic region in the sample propulsion channel prevents sample fluid from entering this channel. Blocking off the end of the sample loading channel 122, or keeping the syringe attached to this channel, another syringe is used to inject a driving fluid into the sample propulsion channel 124 so as to propel 10 nl of sample from the loading chamber 116 through the sample transfer channel 118 and into the first reaction chamber 110, where the desired reactions between the sample and the crystallised reagents is allowed to occur, at a desired temperature.

The contents of the first reaction chamber 110 are then propelled, again using the syringe attached to the end of the sample propulsion channel 124, into the intermediate channel 114 which has a volume slightly greater than the 10 nl of each of the reaction chambers so as to prevent intermixing between the reaction chambers. A 10 nl volume from the intermediate channel is then propelled into the second reaction chamber 112 and the desired reaction allowed to occur between the sample and the reagents therein at the required temperature. Detection of fluorescence or other physical parameters in the second reaction chamber may be carried out in any suitable manner, as described elsewhere in this document. Finally, the sample can be flushed from the system through the exhaust channel.

If the reagents in the reaction chambers are not dessicated but remain in a fluid form, then a slightly different sequence to that described above is required. To ensure sufficient volume in each reaction chamber for the samples and reagents, a reduced volume of reagent fluid should be loaded into each reaction chamber, and it may be desirable to carry out loading of reagents into reaction chambers after the loading of the sample fluid. The first and second reagent channels illustrated in figure 3 connect to the sample transfer channel 118 and the intermediate channel 114 respectively at locations adjacent to the respective reaction chambers, so that effective mixing occurs when a reagent is added to the sample already present in a reaction chamber.

It may be desirable to ensure that the temperature of each reaction chambers is raised to the appropriate operating levels before fluids are introduced, and that the temperatures of other parts of the lumens are stable. Temporal variations in lumen temperature when air is present may lead to unpredictable fluid movements as air thermally expands or contracts.

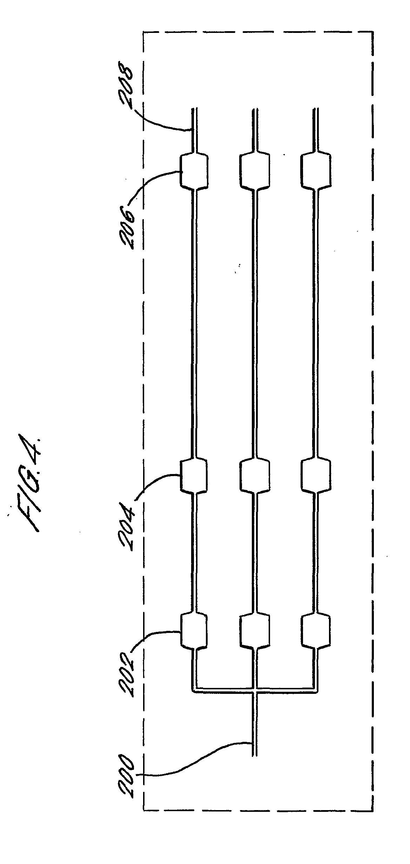

Referring now to figure 4 there is shown in plan view the fluid carrying lumens of a device according to a third preferred embodiment of the invention. Other aspects of the third embodiment, such as general methods of construction, reaction chamber temperature control apparatus, inlet and outlet ports, and illumination and fluoresence detection apparatus are not shown, but may be provided as already described above in respect of the first and second preferred embodiments.

The third embodiment comprises a single input channel 200 which splits into three branches which end in first, second and third parallel systems each comprising a first reagent loading chamber 202, a first reaction chamber 204 and a second reaction chamber 206, connected in sequence. An exhaust channel 208 vents each second reaction chamber 206. Devices constructed according to the third embodiment may be used to carry out multiple sets of reactions in parallel, using a fluid sample introduced via the common input channel 200.

Although the preferred embodiments have been described as constructed using a glass top plate bonded to an etched or otherwise machined silicon substrate, clearly a variety of other construction methods and materials could be used to carry out the invention. For example, chambers and/or other lumens could be created or made larger by forming recesses in the top plate.

A microfabricated reaction chamber device embodying the invention may form an integral part of a larger microfabricated analysis device constructed as a single unit and containing, for example, apparatus for carrying out various sample preparation steps, and containing the various reagents required to carry out the sample preparation steps. Such a microfabricated analysis device could contain some or all of the control and data analysis circuitry required for its operation.

The microfabricated reaction chamber device or larger microfabricated analysis device described above may be designed for installation within a larger analysis unit. Such an analysis unit would typically provide electrical connections to the various active components and sensors of the device, and could provide some or all of the required control and sensor circuitry. The analysis unit could also provide some or all of the optical system, and means for pumping the sample through the reaction chambers. The unit could also provide DNA/RNA sample preparation equipment, although, as mentioned above, some or all of the sample preparation may be carried out in an integral device of which the microfabricated reaction chamber device forms a part. Microfabricated reaction chamber devices embodying the invention may be thereby manufactured for once only use, being installed in an analysis unit when needed and disposed of after use. The larger multi-use analysis unit could conveniently contain sufficient data handling and display equipment to provide a useful hand held analysis device.

### Example

The following Example describes real-time NASBA amplification reactions carried out on 10 nl and 50 nl microchips, or in 0.5 ml and 5 ml capillary tubes.

### Microfabriacted Reaction Chamber System

With reference to Figure 3, a silicon wafer with dry etched microstructure was provided and anodically bonded to a pyrex wafer to thereby define enclosed first and second chambers and associated channels. The glass wafer allowed the reagents to be optically excited and detected through the glass. The silicon wafer enabled electronic components to be integrated by techniques conventional in the semiconductor fabrication art.

The protocol for loading the sample into the chip used a sequencing pipette tip with a 190 µm outer diameter, for loading the reagents. Two different designs were made. The first design included holes etched directly into the channels from the side. Etching openings in the silicon into the channels from the side eliminates the need for fabricating holes in the glass wafer. This process may be preformed simultaneously with the other process steps. The second design employed a tapered hold machined in the 525 µm thick pyrex glass. Holes may be made in pyrex glass by etching, powder blasting, or ultrasonic drilling. Powder blasting is preferred. This is a wafer process and all the holes on one wafer are processed simultaneously.

Sample loading was done manually by inserting disposable, flexible polycarbonate tips with precision tapering into the holes. The samples were injected with a syringe. A rig with five horizontally-mounted syringes was made. The tip positions coincide with the holes on the chip. Micrometer screws were mounted on the syringes in order to control the volume accurately. This method is capable of moving nanoliter volumes of fluids in a controlled fashion.

The channels in the silicon wafer were defined by dry etching, which allowed the microfluidic design to include bends and rounded chambers (see Figure 3). The purpose of the first chamber is to load a defined volume. It is then transported through the chip with air pressure as a liquid plug. Preloaded enzymes are mixed together with the solution in the chamber to the right (see Figure 3). Syringes with micrometer screws are used to control the movement of the liquid plug through the system.

The NASBA process includes two accurately controlled heating steps at 65 and 41 °C. The latter preferably does not exceed 41.5 °C at any point, as this can result in degradation of the reagents. The chip consisted of two silicon blocks connected together by a thin bridge with a channel. Heat can only flow though the bridge of the pyrex glass. Integrated heating elements with temperature sensors may be designed into the mask layout or, alternatively, heating may be performed externally.

The pyrex wafer sealing the channels and chambers is transparent to optical light, and does not fluoresce. Blue light emitted from a 488 nm diode is filtered and focussed into the reaction chamber where it excites the fluorophores. Green fluorescent light, with a wavelength of 518 nm is emitted from the fluorophores, collected by a lens, and guided through filters and onto the detector. The surface in the reaction chamber had a roughness of less than 1/10th of the wavelength of the light. Alcatel performed the Reactive-Ion-Etching (RIE) of the channels and the chambers, and obtained a surface quality with a roughness of less than 40 nm.

The temperature control of the blocks was done externally in the sample holder. Two aluminium blocks were sited on top of Peltier elements. In each block a thermocouple was provided for measuring the temperature of the block with a feedback circuit to the Peltier elements. Both the thermocouple and the sample were attached with thermal grease to maximize the temperature accuracy and stability of the system. Temperature specifications were 41 and 65 °C ± 0.5 °C. The temperature system incorporated digital PID controllers for regulating the temperature. The thermocouples were calibrated by two different methods: (i) a commercial FLUKE temperature calibration apparatus with thermocouples; and (ii) platinum resistance sensors.

The NASBA process activates fluorescent markers, which can be excited and detected optically. An optical system was made for measuring these activated fluorophores. Specifications were: Excitation at 494 nm; and Detection at 518 nm. Data were recorded during the application process. Because the fluorophores get bleached when exposed to blue light, there is a trade-off between the number of measurements and bleaching.

The optical geometry comprised a blue light LED in conjunction with a bandwidth filter for shaping up of the spectral properties. A lens was used to focus the light onto the reaction chamber. Additional lenses collect the fluorescent light from the fluorophores, and guide it to a dichroic beam splitter. The latter projects the light onto a filter, which sits in front of a detector.

### Materials

### Amplification 1:-

Target Oligonucleotide Psek2: 
5'GATTAGACATTTCAGCATACGCATAATCGGCCGGCTTCGCCTAGGCATATCC
TTTGCATGCTACTATATGGACGATACGACCAAATGCCAGTCAGATAGCACAGT
AGCAGCGATTAA (SEQ ID NO:1)
Primer 1:
   5' AATTCTAATACGACTCACTATAGGGAGAAGGGCTGCTACTGTGCTATCTGA (SEQ ID NO:2)
Primer 2:
   5' GACATTTCAGCATACGCATA (SEQ ID NO:3)
Probe:
   5'(FAM)-ATCCTTTGCATGCTACTATA-(DABCYL) (SEQ ID NO:4)
Amplification 2:-
   Target *Staphylococcus epidermidis*: (gene: sdrG, GenBank Accession number AF245042)
   Primer 1:
      pos 584
      5' AATTCTAATACGACTCACTATAGGGAGAAGGGGATTCAGTGTTACTCTCTA (SEQ ID NO:5)
   Primer 2:
      pos 390
      5' GATGCAAGGTCGCATATGAGACAAAAGACCCCTCAAGATA (SEQ ID NO:6)
   Probe:
      pos 475
      5'(FAM)-CCGTCGTACTGCCCAACAACCATCTCCGACGG-(DABCYL) (SEQ ID NO:7)
FAM = carboxyfluorescein
DABCYL = 4-(4-Dimethylaminophenylazo)benzoyl

### Methods:

SIGMACOTE™ (from Sigma-Aldrich Co, Ltd) was used to coat the walls of the chips and capillary tubes before the experiments.

The glass surfaces in the microchip and capillary tubes to be coated were clean and dry.

The glass surface in the microchambers and capillary tubes were covered with undiluted SIGMACOTE™. The reaction is almost instantaneous. Any excess SIGMACOTE™ solution was allowed to evaporate before use.

The two Peltier elements (Marlow Industries M1013T ) in the optical detection system were calibrated to 41.0°C before use with a platinum electrode and a Hewlett Packard 34401A multimeter.

NASBA reagents were prepared according to the application manual supplied with the NucliSens™ Basic Kit supplied by Organon Teknika, as follows:
Preparation of a mastermix:
   1. All reagents were equilibrated to room temperature.
   2. Primers were diluted to a concentration of 10 mM and a primer mix prepared by mixing equal volumes of the first and second primers.
   3. 80 ml of NucliSens reagent sphere diluent was added to the reagent sphere.
   4. 14 ml of KC1 stock solution was mixed with 13.5 ml NASBA water.
   5. The KCl/water mixture of step 4 was added to the reconstituted reagent sphere solution of step 3.
   6. 10 ml of the primer mix was added to the solution of step 5.
   7. 2.5 ml of the molecular beacon probe was added to the solution of step 6 to obtain a mastermix.
   8. The mastermix so-prepared was used within 30 minutes.
Amplification procedure:
   1. 5 ml of the target nucleic acid (sample, Psek2 oligonucleotide or *Staphylococcus* epidermidis genomic DNA) was pipetted into a fresh test tube.
   2. 10 ml of the target RNA-specific primer solution was added.
   3. Test tubes were incubated for 5 ± 1 minutes at 65 ± 1°C.
   4. Test tubes were cooled at 41 ± 0,5°C for 5 ± 1 minutes.
   5. 5ml of enzymes solution was added and the contents of the test tube mixed.
Chips:
   i. A syringe with a sequencing pipet tip (O. D. 0.19 mm) was used to add the sample to the 150-430 mm wide holes in the upper glass of the chip and into reaction chamber number 3 were the measurements were done. The whole chip was filled with the reaction mixture. Wax was used to seal the holes.
   ii. The chip was then placed into a cavity in the optical detection system. Adjustments for accurate positions in the reaction chamber (10 or 50 nl) were made for each individual measurement. Excitation at 494 nm was used for the positioning. (LED from ELFA (Article no. 7500424))
   iii. Measurements were made using periodic measurement; 1 second measurement and 10 second pause for approximately 2 hours. The fluorescence detection was measured at 525 nm. (Detector Photo-Multiplier Tube, H5784 from Hamamatsu)
Capillary tubes 0.5 and 5 ml:
   i. A syringe with a sequencing pipet tip (O. D. 0.19 mm) was used to add the sample to capillary tube. Wax was used to seal the holes.
   ii. The chip was then placed into the optical detection system. Adjustments for accurate positions were made for each individual measurement. Excitation at 494 nm was used for the positioning (LED from ELFA (Article no. 7500424)).
   iii. Measurements were made using periodic measurement; 1 s measurement and 10 s pause for approximately 2 hours. The fluorescence detection was measured at 525 nm. (Detector Photo-Multiplier Tube, H5784 from Hamamatsu).

The present intention provides a microfabricated reaction chamber system and method for real-time nucleic acid sequence amplification and detection. NASBA reactions can be performed in microscopic systems, with the added advantage of reduced reaction times.

The present invention will now be described further, by way of example, with reference to the following numbered clauses.
1. A microfabricated reaction chamber system for carrying out a nucleic acid sequence amplification and detection process on a nucleic acid sample, the process comprising at least first and second process steps using first and second reagents, the device comprising: an inlet port; a first reaction chamber in communication with the inlet port, for carrying out the first process step; a second reaction chamber in communication with the first reaction chamber, for carrying out the second process step; and an outlet port in communication with the second reaction chamber.
2. A system of clause 1 wherein a sample loading chamber is provided between the inlet port and the first reaction chamber.
3. A system of clause claim 1 or claim 2 wherein the first reaction chamber is preloaded with the first reagent, and the second reaction chamber is preloaded with the second reagent.
4. A system of any preceding clause wherein a first temperature controller is provided to control the temperature of the first process step and a second temperature controller is provided to control the temperature of the second process step.
5. A system of clause 4 wherein the first temperature controller is arranged such that the first process step is carried out at a first temperature, and the second temperature controlled is arranged such that the second process step is carried out at a second temperature that is lower than the first temperature.
6. A system of clause 4 or 5 wherein the first temperature controller comprises a first temperature sensor positioned adjacent to the first reaction chamber and the second temperature controller comprises a second temperature sensor positioned adjacent to the second reaction chamber.
7. A system of any preceding clause wherein the first temperature controller comprises a first controllable electric heat source positioned adjacent to the first reaction chamber and the second temperature controller comprises a second controllable electric heat source positioned adjacent to the second reaction chamber.
8. A system of any preceding clause wherein the outlet port is provided with a pressure control valve or a flow control valve.
9. A system of any preceding clause further comprising at least one optical source arranged for exciting fluorescence in material contained within the second reaction chamber, and at least one optical detector, arranged to detect said fluorescence.
10. A system of clause 9 wherein the optical source is provided by one or more light emitting diodes.
11. A system of clause 9 wherein the optical detector comprises at least one avalanche photodiode.
12. A system of clause 9 wherein the optical detector comprises at least one photomultiplier tube.
13. A system of clause 9 wherein a bandpass filter is provided to filter the light impinging on the detector.
14. A system of clause 9 wherein a confocal microscope is provided to direct said fluorescence onto said detector.
15. A system of clause 9 wherein a micro lens is provided to direct said fluorescence onto said detector.
16. An integrally microfabricated reaction chamber system comprising a plurality of sets of interconnected first and second reaction chambers of any preceding clause.
17. A system of clause 16 wherein each of the plurality of sets of interconnected first and second reaction chambers is provided with a separate outlet port.
18. A system of clause 16 or 17 wherein the plurality of first reaction chambers are in communication with a common inlet port.
19. A system of clause 18 wherein the common inlet port is provided with a micro pump to pass the sample into the plurality of sets of first and second interconnected reaction chambers.
20. A system of any preceding clause further comprising a substrate capped with an optically transmitting top plate, each reaction chamber being defined by a recess in a surface of the substrate and by the adjacent surface of the top plate.
21. An apparatus for the analysis of biological and/or environmental samples, the apparatus comprising a system as defined in any one of the preceding clauses.
22. An assay kit for the analysis of biological and/or environmental samples, the kit comprising a system as defined in any one of clauses 1 to 20 and means for contacting the sample with the device.
23. An apparatus of clause 21 or an assay kit of clause 22 which is disposable.
24. A method of carrying out a nucleic acid sequence amplification and detection process on a nucleic acid sample in a microfabricated reaction chamber system, the process comprising at least a first process step using a first reagent and a second process step using a second reagent, the method comprising the steps of: passing the sample into a first reaction chamber and mixing with the first reagent; retaining the sample in the first reaction chamber for a first interval to carry out the first process step ; passing the sample from the first reaction chamber into a second reaction chamber and mixing with the second reagent; retaining the sample in the second reaction chamber for a second interval to carry out the second process step; and during the second interval, measuring a physical phenomenon indicative of the presence of the target nucleic acid sequence.
25. The method of clause 24, wherein the first reagent is preloaded in the first reaction chamber, and wherein the second reagent is preloaded in the second reaction chamber.
26. The method of clause 24 or 25 further comprising the steps of: maintaining the contents of the first reaction chamber at a first temperature during the first interval; maintaining the contents of the second reaction chamber at a second temperature during the second interval; the first temperature being sufficiently high to denature the second reagent.
27. A microfabricated reaction chamber system for carrying out a nucleic acid sequence amplification and detection process on a nucleic acid sample in a single reaction chamber, the device comprising: a reaction chamber having an inlet port and an outlet port and at least one wall that comprises an optically transparent material; means for heating a sample contained the reaction chamber to a temperature of ≤ 41.5 C (preferably from 40.5 to 41.5 C); at least one optical source arranged adjacent said at least one wall for exciting fluorescence in a sample contained in the reaction chamber; and at least one optical detector, arranged to detect said fluorescence through said at least one wall.
28. A method of carrying out a nucleic acid sequence amplification and detection process on a nucleic acid sample in a microfabricated reaction chamber system comprising a single chamber as defined in clause 27, the method comprising:
   assembling a reaction mixture in the single chamber, said mixture comprising the nucleic acid sample, NASBA primers, enzymes for carrying out a NASBA reaction, ribonucleoside and deoxyribonucleoside triphosphates, and molecular beacon probe oligonucleotide; heating said mixture contained in said reaction chamber to a temperature of ≤ 41.5 C (preferably from 40.5 to 41.5 C);
   exciting fluorescence in said mixture contained in said reaction chamber by means of said at least one optical source; and detecting fluorescence in said mixture through said at least one wall by means of said at least one optical detector.

## Claims

1. A microfabricated reaction chamber system for carrying out a nucleic acid sequence amplification and detection process on a nucleic acid sample, the process comprising at least first and second process steps using first and second reagents, the device comprising:
an inlet port;
a first reaction chamber in communication with the inlet port, for carrying out the first process step;
a second reaction chamber in communication with the first reaction chamber, for carrying out the second process step; and
an outlet port in communication with the second reaction chamber.

2. A system as claimed in claim 1, wherein a sample loading chamber is provided between the inlet port and the first reaction chamber.

3. A system as claimed in either claim 1 or claim 2, wherein the first reaction chamber is preloaded with the first reagent, and the second reaction chamber is preloaded with the second reagent.

4. A system as claimed in claim 3, wherein the first reaction chamber is preloaded with NASBA buffer and primer mix solution.

5. A system as claimed in claim 3 or claim 4, wherein the second reaction chamber is preloaded with enzymes for carrying out nucleic acid amplification, said enzymes including reverse transcriptase, T7 RNA polymerase and RNAse H.

6. A system as claimed in any preceding claim, wherein a first temperature controller is provided to control the temperature of the first process step, and a second temperature controller is provided to control the temperature of the second process step, and wherein the first temperature controller is preferably arranged such that the first process step is carried out at a first temperature, and the second temperature controller is preferably arranged such that the second process step is carried out at a second temperature that is lower than the first temperature.

7. A system as claimed in any one of the preceding claims, wherein the volume of said reaction chambers is ≤500 nl, preferably ≤ 300 nl, more preferably ≤ 150 nl.

8. An integrally microfabricated reaction chamber system comprising a plurality of sets of interconnected first and second reaction chambers as claimed in any one of the preceding claims, and wherein each set of reaction chambers is preferably provided with reagents to detect a different target nucleic acid sequence.

9. An apparatus for the analysis of biological and/or environmental samples, the apparatus comprising a system as defined in any one of the preceding claims.

10. An assay kit for the analysis of biological and/or environmental samples, the kit comprising a system as defined in any one of claims 1 to 8 and means for contacting the sample with the device.

11. A method of carrying out a nucleic acid sequence amplification and detection process on a nucleic acid sample in a microfabricated reaction chamber system, the process comprising at least a first process step using a first reagent and a second process step using a second reagent, the method comprising the steps of:
passing the sample into a first reaction chamber and mixing with the first reagent;
retaining the sample in the first reaction chamber for a first interval to carry out the first process step;
passing the sample from the first reaction chamber into a second reaction chamber and mixing with the second reagent;
retaining the sample in the second reaction chamber for a second interval to carry out the second process step; and
during the second interval, measuring a physical phenomenon indicative of the presence of the target nucleic acid sequence.

12. The method of claim 11, wherein the first reagent is preloaded in the first reaction chamber, and wherein the second reagent is preloaded in the second reaction chamber.

13. A system as claimed in claim 12, wherein the first reaction chamber is preloaded with NASBA buffer and primer mix solution.

14. A system as claimed in claim 12 or claim 13, wherein the second reaction chamber is preloaded with enzymes for carrying out nucleic acid amplification, said enzymes including reverse transcriptase, T7 RNA polymerase and RNAse H.

15. The method of any one of claims 11 to 14, further comprising the steps of:
maintaining the contents of the first reaction chamber at a first temperature during the first interval;
maintaining the contents of the second reaction chamber at a second temperature during the second interval;
the first temperature being sufficiently high to denature the second reagent.


SEQUENCE LISTING

<110>  NORCHIP A/S

<120>  Microfabricated reaction chamber

<130>  NLW/P55512EP00

<160>  7

<170>  PatentIn version 3.5

<210>  1
<211>  118
<212>  DNA
<213>  Artificial

<220>
<223>  Synthetic Oligonucleotide

<400>  1
gattagacat ttcagcatac gcataatcgg ccggcttcgc ctaggcatat cctttgcatg      60

ctactatatg ggacgatacg accaaatgcc agtcagatag cacagtagca gcgattaa       118


<210>  2
<211>  51
<212>  DNA
<213>  Artificial

<220>
<223>  Synthetic Oligonucleotide

<400>  2
aattctaata cgactcacta tagggagaag ggctgctact gtgctatctg a               51


<210>  3
<211>  20
<212>  DNA
<213>  Artificial

<220>
<223>  Synthetic Oligonucleotide

<400>  3
gacatttcag catacgcata                                                  20


<210>  4
<211>  20
<212>  DNA
<213>  Artificial

<220>
<223>  Synthetic Oligonucleotide

<400>  4
atcctttgca tgctactata                                                  20


<210>  5
<211>  51
<212>  DNA
<213>  Artificial

<220>

<223>  Synthetic Oligonucleotide

<400>  5
aattctaata cgactcacta tagggagaag gggattcagt gttactctct a          51


<210>  6
<211>  40
<212>  DNA
<213>  Artificial

<220>
<223>  Synthetic Oligonucleotide

<400>  6
gatgcaaggt cgcatatgag acaaaagacc cctcaagata                       40


<210>  7
<211>  32
<212>  DNA
<213>  Artificial

<220>
<223>  Synthetic Oligonucleotide

<400>  7
ccgtcgtact gcccaacaac catctccgac gg                               32